# EUROPEAN PATENT APPLICATION

(11) **EP 1 462 082 A1**
(43) Date of publication of application: **29.09.2004**
(21) Application number: 02802709.2
(22) Date of filing: 01.11.2002
(51) Int. Cl.: A61K 7/00, A61K 7/06, A61K 7/48

(54) **SKIN LOTION COMPRISING AQUEOUS DISPERSION OF ULTRA-FINE NOBLE METAL PARTICLES**

(30) Priority: 06.11.2001 JP 2001340217
(71) Applicant: Phild Co., Ltd., Kyoto city, Kyoto pref. 604-8152 (JP)
(72) Inventor: HIRATA, Yoshihiro, c/o Phild Co., Ltd., Nagayo-ku, Kyoto city, Kyoto 604-8152 (JP); UEDA, Yoshio, c/o Phild Co., Ltd., Nagayo-ku, Kyoto city, Kyoto 604-8152 (JP); TAKASE, Hiroaki, c/o Phild Co., Ltd., Nagayo-ku, Kyoto city, Kyoto 604-8152 (JP); SUZUKI, Kazuaki, c/o Phild Co., Ltd., Nagayo-ku, Kyoto city, Kyoto 604-8152 (JP)
(74) Representative: VOSSIUS & PARTNER
(86) International application number: PCT/JP2002/011420
(87) International publication number: WO 2003/039494

(57) **Abstract**

Use in cosmetic lotions a dispersion solution of super-fine noble metal particles obtained by burning a mixture gas of oxygen and hydrogen in high-pressure water and using the combustion gas to melt noble metal and thus causing super-fine noble metal particles to micro-disperse in water, wherein such production process is implemented using an apparatus for producing a cosmetic lotion that comprises a pressure-resistant container equipped with a high-pressure water tank, 'an injection nozzle for mixture gas of oxygen and hydrogen, a noble metal bar, an ignition device and a combustion chamber.

## Description

### Field of the Invention

This invention relates to a cosmetic lotion made of high-function water in which super-fine noble metal particles are micro-dispersed, a method and apparatus for producing the same, as well as a cosmetic material containing, as its water content, such high-function water in which super-fine noble metal particles are dispersed.

### Background of the Invention

Noble metals possess excellent gloss and corrosion resistance and are therefore used widely in accessories such as rings, necklaces, bracelets and earrings. Due to their corrosion resistance, heat conductivity and other physical properties noble metals are also utilized broadly in industrial applications such as electrodes and electrical contact materials used in electrical and electronics products and energy systems, and also in articles of daily use such as eyeglass frame and clock casing. The applications of noble metals are expected to grow further.

The conventional technologies applying noble metals, for example silver, include a water filter utilizing the antibacterial properties of silver (Japanese Patent Application Laid-open Nos. 7-303809 and 7-195086), dispersion solution of fine particles offering both antibacterial and antifungal effects (Japanese Patent Application Laid-open No. 7-304616), and composition for anti-electrostatic processing for preventing electrostatic in fabrics and leather products (Japanese Patent Application Laid-open No. 11-172154). A coating solution for shielding solar radiation that utilizes ruthenium, iridium or rhodium oxide particles (Japanese Patent Application Laid-open No. 9-302284) is also known.

In addition, several cosmetic materials that utilize noble metals are also known (Japanese Patent Application Laid-open Nos. 61-30511 and 63-96112 and 2001-122723).

However, Japanese Patent Application Laid-open Nos. 61-30511 and 63-96112 above use noble metals as pigments by utilizing their decorative effect, which is different from utilizing noble metals in the production process as function water or in similar forms. In Japanese Patent Application Laid-open No. 2001-122723 above, a cosmetic material for protecting the skin and activating its keratin layer is proposed, wherein such material is produced by dispersing platinum colloid in a water-based agent and the electric double-layer function of obtained high-density, high-activity platinum colloid is used to improve the skin. However, this technology involves dispersion of platinum colloid via chemical reduction of platinum ions in water, and no technology has been available that melts noble metal in high-pressure water using combustion heat from a mixture gas of oxygen and hydrogen and also allows the obtained high-function water in which super-fine noble' metal particles are dispersed to be used as a cosmetic lotion.

### Disclosure of the Invention

The present invention aims to apply these almost infinite utilities of noble metals in bioactive materials, food materials, medical products, and so on―specifically in the production of cosmetic lotions, etc.

The present invention intends to melt noble metal in high-pressure water using a combustion gas of hydrogen and oxygen and cause the molten noble metal to collide with high-pressure water to produce super-fine noble metal particles, and also allow for use of the obtained high-function water containing micro-dispersed noble metal as a cosmetic lotion. The present invention also intends to provide a method and apparatus for producing the aforementioned cosmetic lotion, as well as a cosmetic material containing the aforementioned cosmetic lotion as an active ingredient that acts upon the skin.

In the context of the present invention, noble metal refers to gold, silver, platinum, ruthenium, rhodium, palladium, osmium or iridium or an alloy thereof, and "noble metal micro-dispersion water" refers to water in which a very small amount of noble metal is dissolved in the state of micro-dispersed super-fine particles.

The basic characteristics of the present invention are explained by (1) through (6) below:
(1) A cosmetic lotion comprising noble metal micro-dispersion water in which super-fine noble metal particles are dispersed.
(2) A method for producing a cosmetic lotion in which super-fine noble metal particles are dispersed, wherein a mixture gas of oxygen and hydrogen is burned in high-pressure water and the obtained combustion gas is used to melt noble metal, thereby allowing super-fine noble metal particles to micro-disperse in water.
(3) An apparatus for producing a cosmetic lotion, comprising a pressure-resistant container equipped with a high-pressure water tank, an injection nozzle for mixture gas of oxygen and hydrogen, a noble metal bar, an ignition device and a combustion chamber.
(4) The apparatus for producing a cosmetic lotion as described in Claim 3, wherein a water electrolyzer for producing a mixture gas of oxygen and hydrogen is attached as an adjunct.
(5) A cosmetic material containing, as its water content, noble metal dispersion water in which super-fine noble metal particles are dispersed.
(6) The cosmetic material described in (5) above, which is a creamy soap, cosmetic lotion containing medicament, milky lotion, cream, shampoo, rinse, hair tonic or hair cream.

Here, a cosmetic lotion obtained by the present invention, made of noble metal micro-dispersion water in which super-fine noble metal particles are dispersed, is specifically a skin lotion or other cosmetic lotion that contains the aforementioned micro-dispersion water of super-fine noble metal particles as the main ingredient and also has coloring and aromatic agents added as necessary. A cosmetic material is an aqueous solution, milky lotion or cream in which a medicament acting upon the skin or hair is dispersed, and specific examples of such cosmetic material include a cosmetic lotion, milky lotion, cream, shampoo, rinse, hair tonic, hair cream or hair growth stimulant/hair tonic containing creamy soap or medicament.

Generally in an aqueous dispersion solution or mixed solution in which super-fine particles of noble metals are simply dispersed, the noble metal settles and separates within a short period of time. The most significant feature of the noble metal micro-dispersion water obtained by the present invention is that the noble metal that has been melted by combustion heat disperses in high-pressure water as super-fine particles. Thus obtained micro-dispersion water does not cause the noble metal to settle or otherwise separate from water over a long storage period, but allow it to remain micro-dispersed in a stable condition instead. The noble metal micro-dispersion water obtained by the present invention therefore exhibits the remarkable effect of providing a bioactive cosmetic lotion or cosmetic material through an interaction of water molecules and super-fine noble metal particles, which could not be expected from the conventional technologies.

In general, healthy skin retains 10 to 20% of water in its keratin layer to maintain the skin's suppleness. If the water content in the keratin layer drops to 10% or less, the skin will become dry, and dullness, rough skin, generation of white powder comprising separated dead skin cells, or chapped skin will result. To treat such skin, water and oil must be replenished externally to restore a normal moisture/oil balance in the skin. On the other hand, hair is damaged by external irritations such as friction, heat from the sun, perm chemicals and inappropriate cuts, as well as by malnutrition caused by an unbalanced diet or drastic weight loss. Since damaged hair has no self-repairing ability, it must be protected externally to prevent further damage. Water and oil are two important constituents of the skin and hair, and cosmetic lotions, treatment agents and hair growth stimulants are used to replenish water and oil in our skin and hair.

In order to maintain supple skin, the skin's keratin layer retains the water secreted from sweat glands or absorbed through the skin, while evaporation of the retained water is prevented by the sebum cutaneum. Water is also absorbed into the hair cortex, which is a component of hair, and adds moisture and a supple look to the hair while making the hair more flexible so as to let it bounce as the body moves. Oil forms an oil film over the hair cuticle and adds shine and gloss to the hair while minimizing friction and preventing damage to the hair cuticle.

The noble metal micro-dispersion water obtained by the present invention has been confirmed to provide excellent skin activation effects such as repairing small cuts in the skin caused by razor blades, etc., as well as remarkable repair effects on damaged hairs such as those having nicks on the surface, which are not available with the cosmetic lotions or treatment agents currently available in the market.

In addition to the aforementioned cosmetic lotion or cosmetic material, the aforementioned noble metal micro-dispersion water obtained by the present invention can also be utilized in health products such as motor-function enhancing creams, medical products such as antibacterial agents, UV-protection products, healthy drinking water, health supplements, food preservatives, freshness-keeping agents for food, insect repellents, deodorants, and so on.

The mechanism as to why such noble metal micro-dispersion water in which super-fine noble metal particles are dispersed, as obtained by the present invention, provides excellent bioactive functions is not yet clear. The inventors are working diligently to find scientific explanations for these functions.

### Brief Description of the Drawings

Fig. 1: Production flow chart of a cosmetic lotion made of noble metal micro-dispersion water as proposed by the present invention
Fig. 2: Apparatus for producing a cosmetic lotion made of noble metal micro-dispersion water as proposed by the present invention
Fig. 3: Table showing the analysis test results of noble metal (gold) micro-dispersion water as proposed by the present invention

### Description of the Symbols

1: Production apparatus for noble metal micro-dispersion water as proposed by the present invention
2: Pressure-resistant container for producing noble metal micro-dispersion water
3: Electrolyzer/material gas generator
5: High-pressure water tank
6: Combustion chamber
7: Pressure regulating valve
8: Outlet for noble metal micro-dispersion water
9: High-pressure water
10: Titanium metal bar
11: Ignition device
12: Molten titanium metal
13: Feed cylinder
14: Mixture gas injection nozzle
16: Hydrogen feed channel
17: Oxygen feed channel
18: Cathode
18': Anode
19: Partition
20: Water

### Best Mode for Carrying Out the Invention

A cosmetic material made of noble metal micro-dispersion water as obtained by the present invention or cosmetic lotion containing noble metal micro-dispersion water as its water content is a new product not heretofore available. The present invention also provides a new method and apparatus for producing a cosmetic material made of water that contains noble metal as super-fine particles, wherein the production of such noble metal micro-dispersion water is based on melting noble metal not through general methods that are commonly used to melt metal, but by using heat generated from burning oxygen and hydrogen and causing the obtained molten noble metal to disperse in high-pressure water.

Specifically, after studying ways to efficiently and economically produce a cosmetic lotion made of noble metal micro-dispersion water, as well as ways to apply such micro-dispersion water to bioactivation purposes, the inventors conceptualized a method to burn hydrogen and oxygen, insert a bar made of pure noble metal into the combustion atmosphere to heat the noble metal bar, and cause the obtained molten noble metal to collide with high-pressure water and disperse in water as super-fine particles, wherein such combustion of hydrogen and oxygen is caused in high-pressure water so as not to produce substances other than water and noble metal.

The method proposed by the present invention requires that the amounts of hydrogen and oxygen to be burned, reaction pressure and feed rate of noble metal be controlled. In the aforementioned production method, an appropriate filter system is also necessary because not only super-fine noble metal particles but also a trace amount of fine particles of noble metal oxide will be produced in water.

A method for producing a cosmetic material made of noble metal micro-dispersion water as obtained by the present invention in high-pressure water through the aforementioned method, as well as an apparatus to embody this method, are explained according to the drawings.

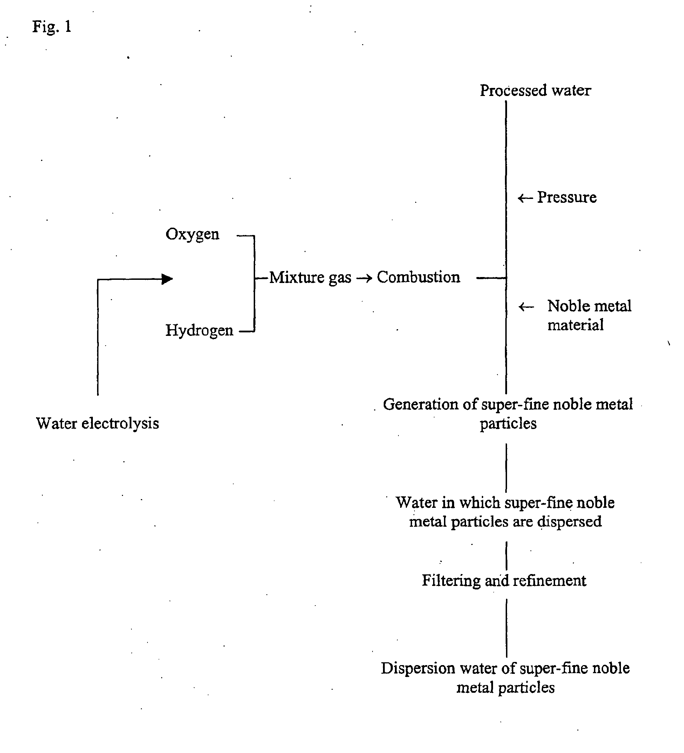

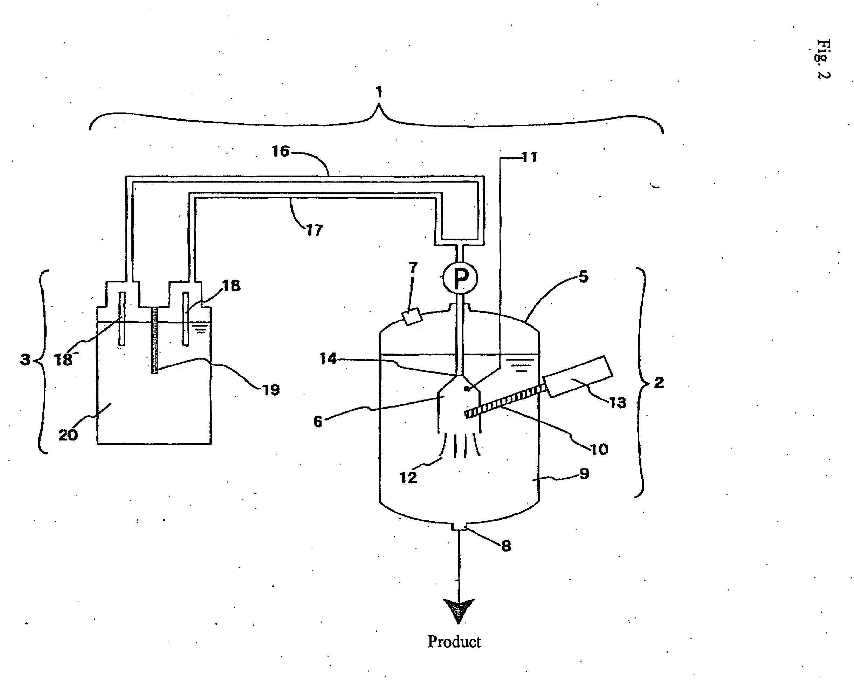

Fig. 1 is a production flow chart of a cosmetic lotion made of noble metal micro-dispersion water as proposed by the present invention, while Fig. 2 shows an apparatus for producing a cosmetic lotion made of noble metal micro-dispersion water as proposed by the present invention.

The production apparatus for noble metal micro-dispersion water as proposed by the present invention (1), as shown in Fig. 2, comprises a pressure-resistant container (2) for producing high-pressure water in which molten noble metal is dispersed, an electrolyzer/material gas generator (3) and a filter system to filter noble metal micro-dispersion water (not illustrated).

The basic structure of the pressure-resistant container (2) proposed by the present invention comprises a production apparatus for noble metal micro-dispersion water in which super-fine noble metal particles are dispersed, wherein such apparatus comprising a high-pressure water tank (5), an injection nozzle for mixture gas of oxygen and hydrogen (14), a combustion chamber (6) and a noble metal bar (10). A water electrolyzer (3) for supplying material hydrogen and oxygen and a filter system that filters produced noble metal micro-dispersion water are attached as adjuncts.

The pressure-resistant tank (2) proposed by the present invention comprises the high-pressure water tank (5) made of metal, or preferably steel. In this high-pressure water tank (5), a mixture gas of the hydrogen and oxygen generated by the electrolyzer (3) and supplied through a hydrogen feed channel (16) and oxygen feed channel (17) is injected at high pressure from an injection nozzle (14) into the combustion chamber (6). The noble metal bar (10) is fed by a feed cylinder (13) into the combustion chamber (6) successively in accordance with the melting amount of noble metal. The mixture gas of hydrogen and oxygen is ignited by an ignition device (11), and molten noble metal is released into high-pressure water (9). The molten noble metal collides with high-pressure water and becomes super-fine particles, and the high-pressure water (9) containing these super-fine noble metal particles is retrieved from the high-pressure water tank via an outlet (8) at the bottom of the tank and then filtered by an appropriate filter system (not illustrated).

As for the material gas generator (3), the hydrogen and oxygen produced by water electrolysis are pure gases, and therefore use of an electrolyzer has the advantage of enabling an efficient supply of combustion gas of oxygen and hydrogen mixed at a theoretical ratio of 1 to 2.

The present invention provides an example of generating hydrogen and oxygen via electrolysis of water (20) in the material gas generator (3), as material gases used in the production of noble metal micro-dispersion water. A cathode and an anode are denoted as (18) and (18'), respectively. Hydrogen and oxygen may also be supplied directly to the high-pressure water tank (5) from respective cylinders.

This apparatus injects hydrogen and oxygen, generated via electrolysis and supplied through the hydrogen feed channel (16) and oxygen feed channel (17), into the combustion chamber (6) from the nozzle (14) using a pump, and causes the mixture gas to burn completely to achieve a state of perfect steam gas of ultrahigh temperature. The pure noble metal bar (10) is inserted into this combustion gas to be heated and melted. The noble metal bar is inserted via the cylinder (13) at a constant rate according to the melting amount. To melt the noble metal bar, the mixture ratio of hydrogen and oxygen must be strictly controlled at 2 to 1. Also, a pressure regulating valve (7) must be provided to regulate the pressure inside the high-pressure water tank.

The molten noble metal (12), heated to high temperature and thus melted in the combustion chamber (6), is released from the combustion chamber (6) into high-pressure water (9), where the noble metal collides with the high-pressure water to become super-fine particles. It is considered that a part of noble metal takes on a crystalline structure in this process.

The produced super-fine noble metal particles in water have very strong hydrophobicity and therefore remain micro-dispersed in water in a stable condition without settling even when a coagulating agent is added.

As for the operation of this apparatus, high-pressure hydrogen and oxygen are injected into the high-pressure water tank (5) from the nozzle (14) using a pump, the gas is ignited by the injection device (11) to achieve a state of perfect steam gas of ultrahigh temperature, and the pure noble metal bar (10) is successively inserted into the combustion gas and melted.

In this apparatus, hydrogen and oxygen must be burned in high-pressure water in impurity-free state so as not to produce substances other than water and super-fine noble metal particles. In addition, the position where the noble metal bar is inserted must be where the mixture gas burns completely to achieve a state of complete steam gas of ultrahigh temperature.

Other characteristics of the present invention include the refining of thus produced noble metal micro-dispersion water, in which fine particles of noble metal oxide are contained in addition to dispersed super-fine noble metal particles, in order to use the water as a material for cosmetic lotions and other cosmetic products as well as health products, food materials, drugs and quasi-drugs. So as not to remove the produced super-fine titanium particles more than necessary, filtering should preferably be implemented not by using ion exchange or reverse-osmosis membrane, but by using the filter system as explained below that can yield noble metal micro-dispersion water suitable for a desired purpose. For example, filtering the high-pressure water discharged from the high-pressure water tank sequentially through hollow-fiber membranes is beneficial in terms of enhancing the characteristics of noble metal micro-dispersion water and extending the filter life, and such filter system will provide noble metal micro-dispersion water that can meet the required standards governing the aforementioned products.

The constituent analysis of noble metal micro-dispersion water as obtained by the present invention found a specified amount of super-fine noble metal particles and a very small amount of noble metal in the high-pressure water.

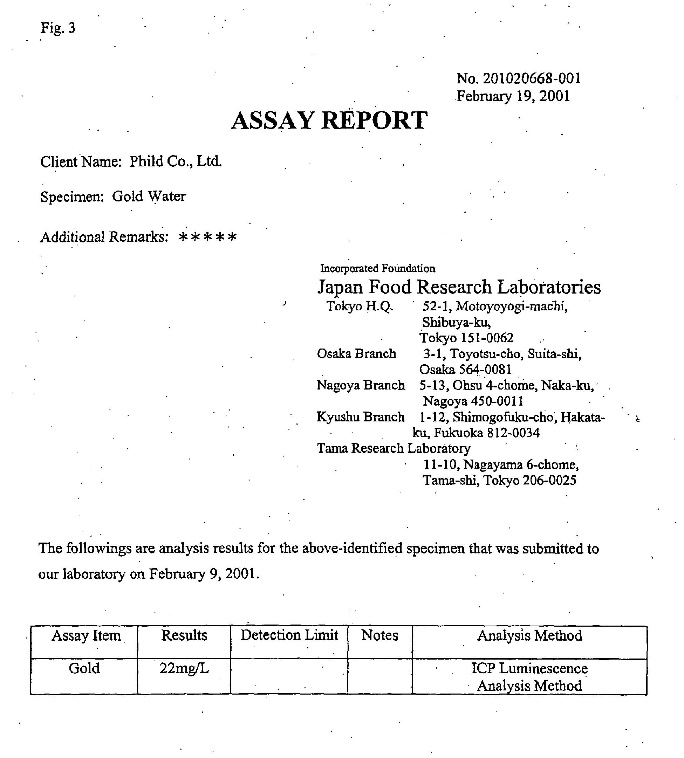

The analysis results of gold micro-dispersion water as obtained by the present invention are shown in Fig. 3.

### Example:

An embodiment of the present invention is explained in details using an example. Note, however, that the present invention is not limited to this example.

Fig. 2 shows an example of the representative apparatus proposed by the present invention, wherein the apparatus comprises a high-pressure water tank (5), an injection nozzle for mixture gas of oxygen and hydrogen (14) and a material gold bar (10) and is used for producing a cosmetic lotion made of gold micro-dispersion water in which super-fine gold particles, generated as a result of collision between molten gold and high-pressure water, are dispersed.

The high-pressure water tank (5) is a metal pressure-resistant tank providing resistance under ultrahigh pressures, wherein a mixture gas of hydrogen and oxygen supplied through the hydrogen feed channel (16) and oxygen feed channel (17) is injected into a combustion chamber (6) through the injection nozzle (14), and the gold bar (10) is fed into the combustion chamber via a cylinder (13). The pressure inside the high-pressure water tank (5) must be controlled using a pressure regulating valve (7). The mixture gas is ignited by an ignition device (11), and the obtained molten gold (12) is released into, and collides with, high-pressure water to become super-fine gold particles. Thus produced gold micro-dispersion water in which super-fine gold particles are micro-dispersed is retrieved from the tank via an outlet (8).

As for the operation of this apparatus, as mentioned above hydrogen and oxygen are fed under high pressure into the high-pressure water tank (5). The mixture gas is injected from the nozzle (14) and ignited by the ignition device (11) to be burned completely to achieve a state of perfect steam gas of ultrahigh temperature. The gold metal bar (10) is inserted into this combustion gas and melted. Gold (12), thus heated to high temperature and melted in the nozzle, is released into, and collides with, high-pressure water to become super-fine gold particles. At this time, a part of gold takes on a crystalline structure, to which gold atoms will rearrange themselves to form particles of near-spherical shape, thus attaining an energetically stable state.

The produced gold micro-dispersion water contains super-fine gold particles that remain in micro-dispersed state without using any active agent. This water is retrieved from the outlet and fed to a filter system as appropriate. The filter system comprises filter membranes of 50 microns, 25 microns, 3 microns, 0.5 micron and 0.1 micron, and by passing the produced water through these filter membranes sequentially a final gold micro-dispersion water containing a specified amount of super-fine gold particles and a very small amount of dissolved gold is obtained.

It is appropriate to adjust the internal pressure of the production tank to approximately 2 atmospheres, feed rate of oxygen and hydrogen mixture gas (1:2) to 5 liters per second (3.5 atmospheres), and feed rate of the gold bar to 0.5 kg over two hours.

In the aforementioned production method, in order to produce one ton of noble metal micro-dispersion water a mixture gas of around 3.5 atmospheres should be injected at approximately 4 to 6 liters per second into the high-pressure water tank containing water pressured to around 1.5 to 2.5 atmospheres. An excessive gas pressure may damage the structural components of the apparatus, while too low a pressure will cause water to enter the combustion chamber and allow the heated/melted noble metal to dissipate into air in air bubbles, thus making less ideal the production condition of micro-dispersion water containing super-fine particles.

### Trial Test of Skin Lotion Made of Gold Micro-Dispersion Water

Ten adult female subjects were instructed to use gold micro-dispersion water in which super-fine gold particles are dispersed, and the effects and efficacies of the treatment were verified.

The subjects participating in the test applied to their face a cosmetic lotion made of the gold micro-dispersion water obtained under the aforementioned condition.

### Use Conditions and Test Results

1. 10 female subjects (The subjects used the water at varying frequencies)
2. Efficacies

| | | | |
|---|---|---|---|
| Skin became suppler | 5 persons | Wrinkles became less | 4 persons |
| Skin became smoother | 8 persons | Wrinkles disappeared | 3 persons |
| Dull skin complexion disappeared | 3 persons | | |

### Trial Test of Hair Tonic/Hair Growth Stimulant Made of Gold Micro-Dispersion Water

The effectiveness, as a hair growth stimulant/hair tonic, of a cosmetic material made of the gold micro-dispersion water obtained in the same manner was tested.

Ten adult male and female subjects were instructed to apply to their hair noble metal micro-dispersion water in which super-fine noble metal particles are dispersed, and the effects and efficacies of the treatment were verified.

The subjects participating in the test used a hair growth stimulant/hair tonic made of the noble metal micro-dispersion water obtained under the aforementioned condition by washing their hair with the water or spraying/applying the water to their hair.

### Use Conditions and Test Results

Use conditions: Washing hair in a basin (3 liters per wash)
: Spraying onto hair with a spray (10 cc per application)
: Applying onto hair using a brush (10 cc per application)
1. 10 male and female subjects (The subjects used the liquid at varying frequencies)
2. Efficacies

| | |
|---|---|
| Hair became less tangling | 5 persons |
| Fallen hair became less | 8 persons |
| Hair became shiner | 7 persons |
| Hair became darker | 1 person |
| Hair became denser | 6 persons |
| Scalp became smoother | 7 persons |
| Dandruff became less | 7 persons |

As evident from the above test results, many of those who used the gold micro-dispersion water obtained by the present invention cited improved skin conditions such as suppler and smoother skin. Also, many of those who washed their hair with the gold micro-dispersion water or applied/sprayed it to their hair cited reduction in fallen hair, increase in hair density, recovery of damaged hair, improved hair condition, smoother scalp, reduced dandruff and other improvements. Therefore, it is found that a cosmetic material made of gold micro-dispersion water as obtained by the present invention would be effective in improving the skin condition and promoting hair growth. The skin activation effects of such water will likely help reduce and prevent skin pigmentations and also prevent aging of the skin, thereby delivering remarkable effects as drugs, cosmetics, and so on.

Although the above example described an example of using gold as the noble metal, tests using other noble metals confirmed equivalent effects.

Based on the confirmed effects of noble metal micro-dispersion water as explained above, use of noble metal micro-dispersion water obtained by the present invention as the water content for various cosmetic materials, instead of normal demineralized water, will likely produce high-function cosmetic materials offering the efficacies of active ingredients contained in the cosmetic material as well as the effects of noble metal micro-dispersion water.

### Industrial Field of Application

The present invention, whose structure is explained above, provides a cosmetic lotion comprising newly developed noble metal micro-dispersion water, as well as its production method and apparatus, and allows for efficient production of a cosmetic lotion whose main ingredient is bioactive noble metal micro-dispersion water containing super-fine noble metal particles in micro-dispersed state. Trial tests also found that cosmetic materials comprising thus obtained noble metal micro-dispersion water are effective in replenishing water in the skin to make it suppler and smoother, and also in repairing damaged hair.

The high-function water obtained by the present invention is also useful as material for various cosmetic products, healthy drinking water, health supplements, food preservatives, freshness-keeping agents for food, insect repellents and deodorants, which suggests beneficial effects of such water in industrial applications.

## Claims

1. A cosmetic lotion comprising noble metal micro-dispersion water in which super-fine noble metal particles are micro-dispersed.

2. A method for producing a cosmetic lotion, comprising:
burning a mixture gas of oxygen and hydrogen in high-pressure water; and
melting noble metal using the obtained combustion gas, thereby allowing super-fine noble metal particles to micro-disperse in water.

3. An apparatus for producing a cosmetic lotion, comprising a pressure-resistant container equipped with a high-pressure water tank, an injection nozzle for mixture gas of oxygen and hydrogen, a noble metal bar, an ignition device and a combustion chamber.

4. The apparatus for producing a cosmetic lotion as described in Claim 3, further comprising a water electrolyzer for producing a mixture gas of oxygen and hydrogen.

5. A cosmetic material containing, as its water content, noble metal dispersion water in which super-fine noble metal particles are micro-dispersed.

6. The cosmetic material described in Claim 5, which is a creamy soap, cosmetic lotion containing medicament, milky lotion, cream, shampoo, rinse, hair tonic or hair cream.
